(19) Europäisches Patentamt
European Patent Office
Office européen des brevets

(11) Veröffentlichungsnummer: **0 007 040**
**B1**

(12) **EUROPÄISCHE PATENTSCHRIFT**

(45) Veröffentlichungstag der Patentschrift: **29.04.81**

(51) Int. Cl.³: **C 07 D 211/40,** C 07 D 211/46

(21) Anmeldenummer: **79102174.4**

(22) Anmeldetag: **29.06.79**

(54) **Herstellung von 1-Desoxy-nojirimycin und N-substituierten Derivaten.**

(30) Priorität: **11.07.78 DE 2830469**

(43) Veröffentlichungstag der Anmeldung: **23.01.80 Patentblatt 80/2**

(45) Bekanntmachung des Hinweises auf die Patenterteilung: **29.04.81 Patentblatt 81/17**

(84) Benannte Vertragsstaaten: **AT BE CH DE FR GB IT NL SE**

(56) Entgegenhaltungen:
**DE-A1-2 738 717**
**DE-A1-2 824 781**
**DE-A1-2 846 118**
**Chemische Berichte, Band 100, Nr. 3, 1967**
**H. Paulsen et al, »Synthese und Reaktionen von Keto-piperidinosen«.**
**Seiten 802 bis 815**
**Chemische Berichte, Band 100, Nr. 8, 1967**
**H. Paulsen et al. »Darstellung von 5-Hydrazino-5-desoxy-D-xylose. Umwandlung in N-Amino-D-xylopiperidinose und ein dimeres Hexahydrotetrazin-Derivat«**
**Seiten 2467 bis 2473**

(73) Patentinhaber: **BAYER AG, Zentralbereich Patente, Marken und Lizenzen, D-5090 Leverkusen 1, Bayerwerk (DE)**

(72) Erfinder: **Stoltefuss, Jürgen, Parkstrasse 20, D-5657 Haan 2 (DE)**

**EP 0 007 040 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

Herstellung von 1-Desoxy-nojirimycin und N-substituierten Derivaten

Die vorliegende Erfindung betrifft ein neues, chemisch eigenartiges Verfahren zur Herstellung der Verbindungen der Formel

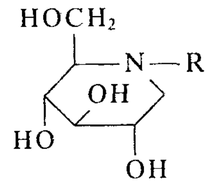

(I)

worin R Wasserstoff, Alkyl, Aralkyl oder Aryl bedeutet.

Es ist bereits bekannt geworden, daß die unter dem Namen 1-Desoxy-nojirimycin bekannte Verbindung der Formel (I; R=H) entweder durch Extraktion von Pflanzen der Moros-Arten gemäß deutscher Offenlegungsschrift 2 656 602 oder mikrobiologisch mit Hilfe von Organismen der Familie Bacillaceae, insbesondere des Stammes DSM 7 gemäß deutscher Offenlegungsschrift 2 658 563 erhalten werden kann. Die Verbindungen der Formel (I) können als Mittel gegen Diabetes, Hyperlipämie und Adipositas verwendet werden.

Außerdem ist bereits bekannt geworden, daß man 1-Desoxynojirimycin durch Hydrierung der wenig stabilen freien Basen (II) oder (III)

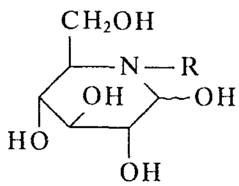

(II)

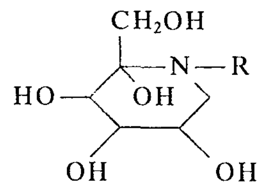

(III)

nach H. Saki und E. Ohki, Chem. Pharm. Bull. 16, 2477 bis 2481 (1968) und H. Paulsen, I. Sangster und H. Heyns, Chem. Ber. 100, 802 bis 815 (1967) herstellen kann.

Die dabei als Ausgangsprodukt verwandte Verbindung (II) wird hergestellt, indem man entweder 5-Amino-5-desoxy-1.2-O-isopropyliden-α-D-glucofuranose (IV) durch 60stündiges Einleiten von Schwefeldioxid in ein stabiles Bisulfit-Addukt (V) überführt, welches dann durch Behandlung mit Bariumhydroxid II ergibt,

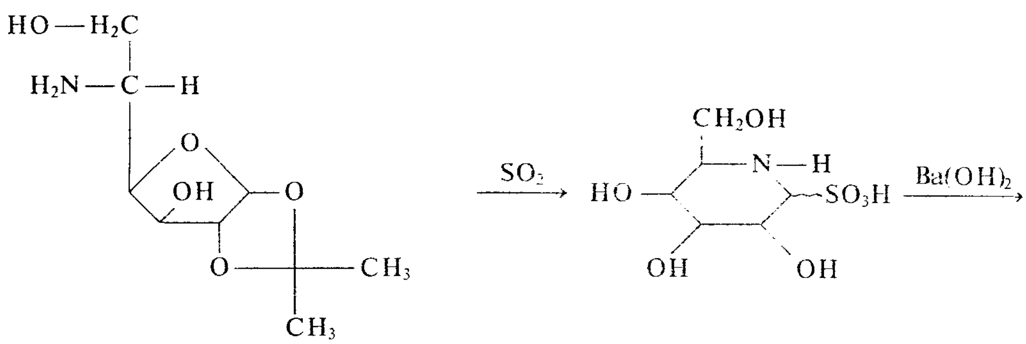


(IV) (V) (II)

(DE-AS 17 68 044)

oder indem man die Verbindung IV in das trifluoracetylierte Derivat VI überführt, welches durch

Verkochen mit verdünnter Salzsäure das Trifluoracetylderivat VII ergibt. Durch anschließende Abspaltung über Anionenaustauscher erhält man (II)

(IV)

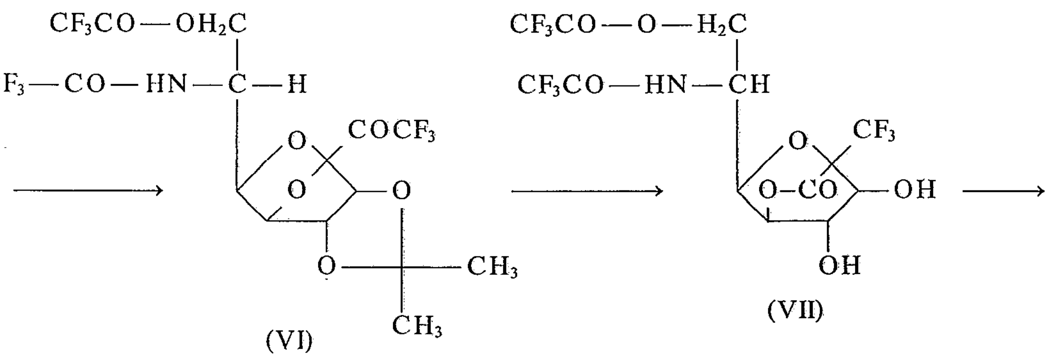

(VI) (VII) (II)

oder man stellt die Verbindung (II) mikrobiologisch her (US-PS 3 998 698).

Die Verbindung (III) gewinnt man aus 6-Amino-2.3-O-isopropyliden-6-desoxy-α-L-sorbofuranose VIII durch Spaltung und anschließende Freisetzung des erhaltenen Hydrochlorides IX durch Chromatographie über einen Anionenaustauscher.

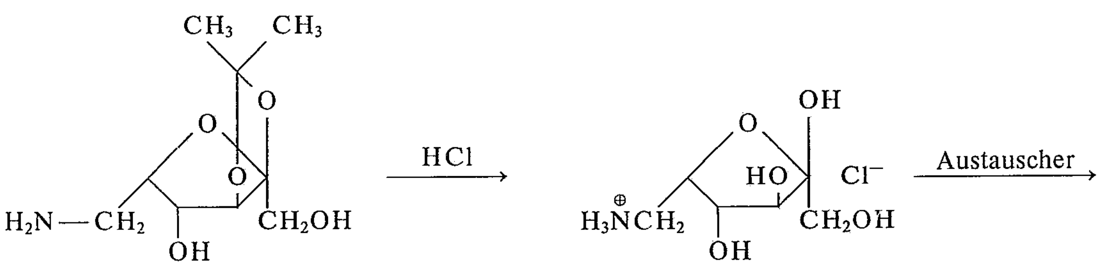


(VIII) (IX) (III)

Die hier aufgeführten Verfahren zur Herstellung von 1-Desoxynojirimycin verlaufen alle über mehrere zeitraubende Stufen, wobei das Durchlaufen der instabilen Verbindungen II und III als Zwischenprodukte zwangsläufig zu Nebenprodukten führen muß.

Außerdem erfordern die bisher bekannten Verfahren zur Herstellung der Verbindung I (R=H) in jedem Fall aufwendige Reinigungsschritte wie Extraktion, Säulenchromatographie oder Chromatographie an Austauschern, die zum Teil auch dadurch notwendig werden, daß die Hydrierung der freien Basen nicht stereospezifisch verläuft.

Es wurde nun überraschend gefunden, daß man die Verbindungen I in ausgezeichneten Ausbeuten erhält, wenn man Verbindungen der allgemeinen Formeln (X) oder (XI),

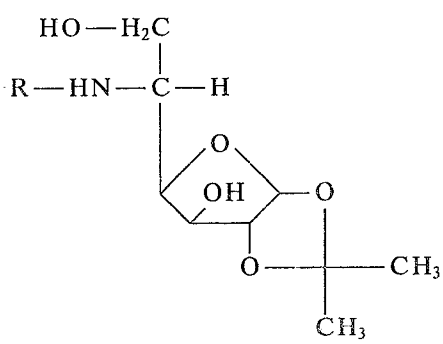

(X)

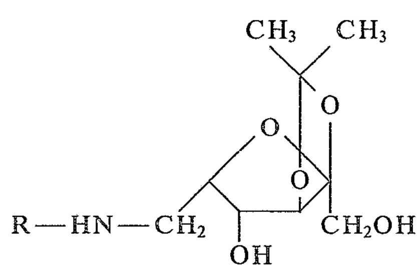

(XI)

in welchen R die oben angegebene Bedeutung hat, durch Behandlung mit Säuren zu Ammoniumsalzen

der vermutlichen Strukturen XII und XIII deblockiert

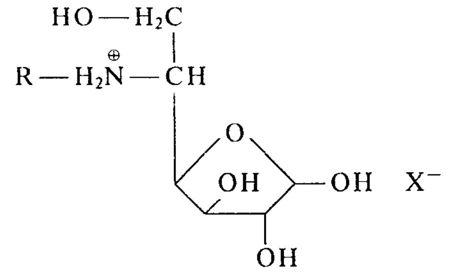

(XII)

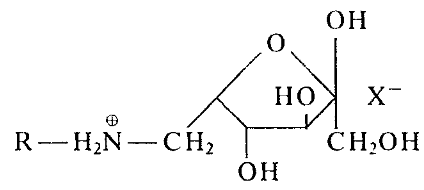

(XIII)

und diese dann entweder isoliert und mit einem geeigneten Wasserstoffdonor hydriert oder in einem Eintopfverfahren die Verbindungen X und XI zuerst durch Behandlung mit Säuren deblockiert und nach gezielter Zugabe von Base direkt hydriert. Darüber hinaus ergibt die Hydrierung der Salze XIII in stereospezifisch ablaufender Reaktion nur die gewünschten Glucoverbindungen.

Es ist als ausgesprochen überraschend zu bezeichnen, daß gemäß der erfindungsgemäßen Umsetzung die Verbindungen I in ausgezeichneten Ausbeuten und stereospezifisch einheitlich erhalten werden, weil man nach dem Stand der Technik erwarten mußte, daß die Deblockierung der Verbindungen X mit Mineralsäuren lediglich zu Pyridinderivaten und die Hydrierung der durch Deblockierung von XI erhaltenen freien Basen zu Gemischen von gluco- und ido-Verbindungen (Paulsen, s. S. 2) führen würde.

Vorzugsweise bedeutet R Wasserstoff, Alkyl mit 1 bis 30, insbesondere 1 bis 18 C-Atomen, gegebenenfalls substituiertes Phenyl, Phenyl-$C_1-C_4$-alkyl oder Naphthyl-$C_1-C_6$-alkyl, wobei als Substituenten von Phenyl und Naphthyl Halogen, insbesondere F, Cl oder Br, $C_1-C_4$-Alkyl, $C_1-C_4$-Alkoxy, Nitro, Amino oder Cyan in Frage kommen.

In ganz besonders bevorzugten Verbindungen der Formel I hat R die Bedeutuing Wasserstoff, $C_1-C_{12}$-Alkyl, insbesondere $C_1-C_4$-Alkyl oder Phenyl.

Die als Ausgangsprodukte verwendeten Verbindungen der Formeln X und XI können nach an sich bekannten Methoden hergestellt werden, indem man z. B. 3-O-Benzyl-6-O-triphenylmethyl-1.2-O-isopropyliden-α-D-xylo-hexofuranos-5-ulose reduktiv aminiert und anschließend die Abspaltung der Trityl- und Benzyl-Gruppe mit Natrium in flüssigem Ammoniak durchführt

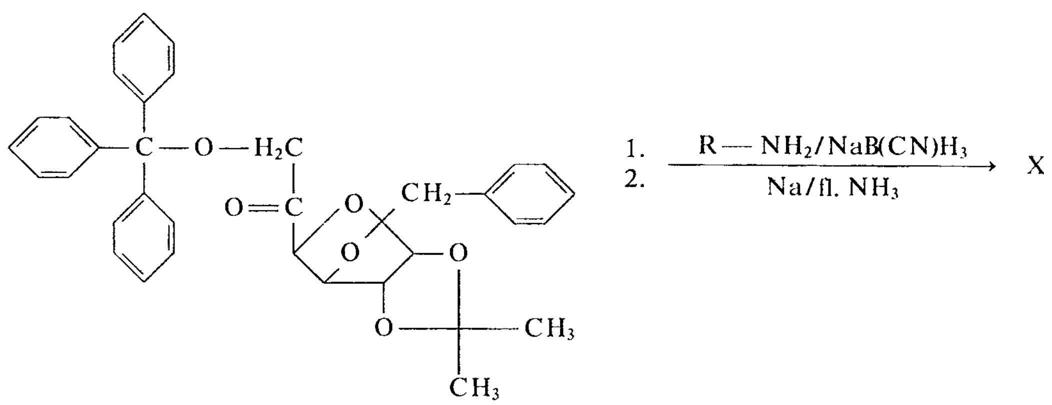


oder indem man z. B. 2.3-O-Isopropyliden-6-O-p-toluolsulfonyl-α-L-sorbofuranose mit Natriumazid umsetzt, anschließend hydriert und eventuell reduktiv alkyliert; oder direkt mit Aminen umsetzt.

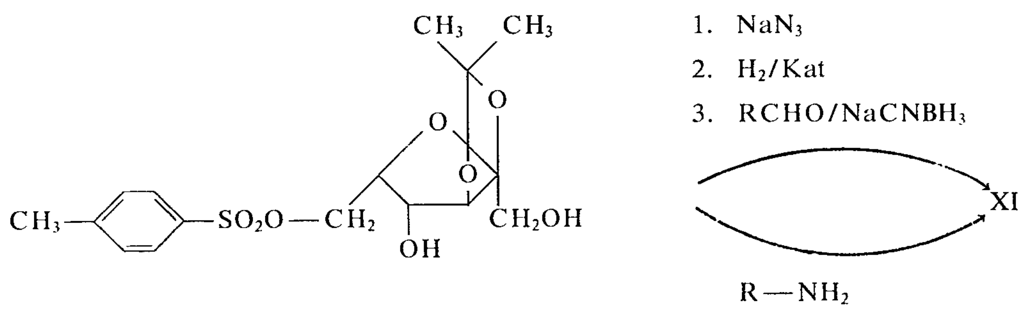

Verwendet man beispielsweise als Ausgangsprodukt 6-Amino-2.3-O-isopropyliden-6-desoxy-α-L-sorbofuranose und als Säure Salzsäure, so kann der Reaktionsablauf durch das folgende Formelschema wiedergegeben werden:

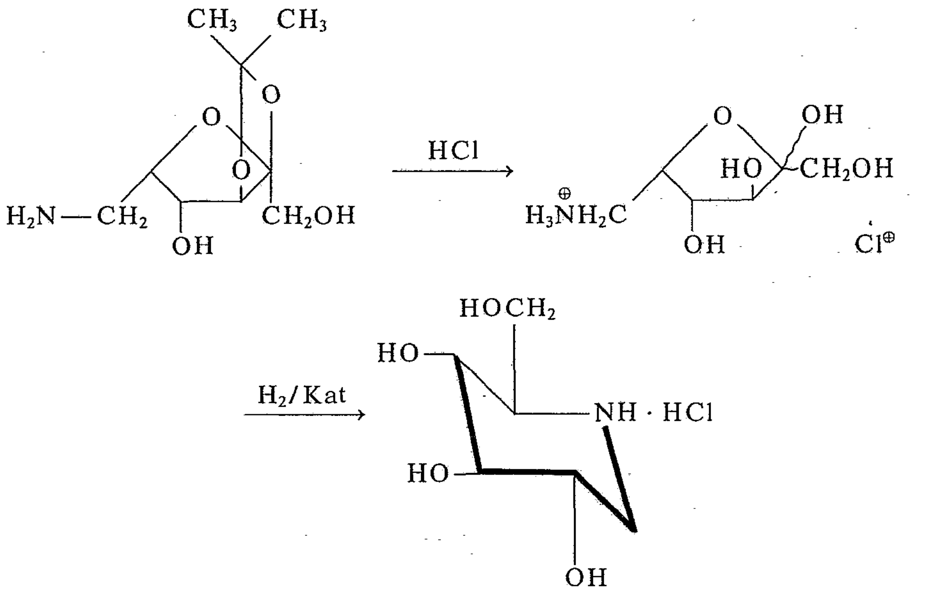


Verwendet man als Ausgangsprodukt 1,2-O-Isopropyliden-5-methylamino-5-desoxy-α-D-glucofuranose, so läßt sich der Reaktionsablauf wie folgt formulieren:

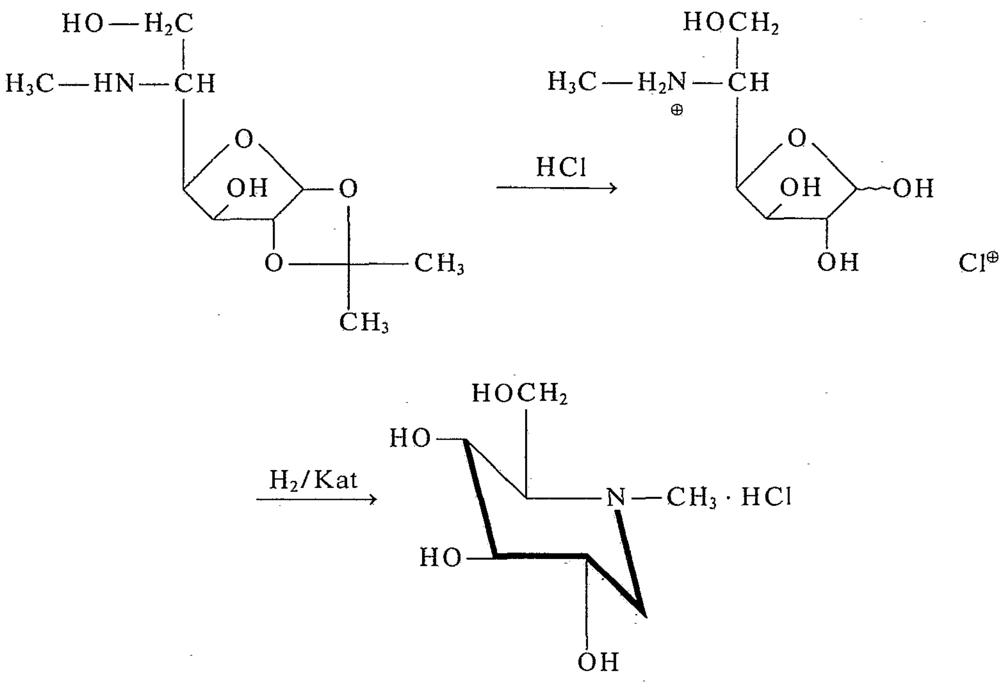


Aus den dabei entstehenden Salzen lassen sich leicht durch Zugabe geeigneter anorganischer oder organischer Basen oder durch Neutralisation mit Anionenaustauschern die freien Verbindungen herstellen.

Die in dem erfindungsgemäßen Verfahren verwendeten Säuren können Mineralsäuren wie Salzsäure oder Schwefelsäure oder auch organische Säuren wie Essigsäure oder Ameisensäure sein, vorzugsweise wird jedoch Salzsäure verwendet, wobei die Konzentration für die Abspaltung der Isopropyliden-Gruppe im weiten Bereich variiert werden kann, vorzugsweise verwendet man 2 N bis 6 N Salzsäure.

Es ist ein wesentlicher Teil dieser Erfindung, daß die Säurekonzentration vor der Hydrierung durch Zugabe von anorganischen oder organischen Basen, vorzugsweise Triäthylamin oder Pyridin, verringert wird.

Man erhält I in großer Reinheit und besonders hoher Ausbeute, wenn für ein Mol zu hydrierende Verbindung 1 bis 1,2-Äquivalent Säure vorhanden ist.

Als Verdünnungsmittel kommen Wasser, niedrige Alkohole und andere protische Lösungsmittel in Frage, vorzugsweise jedoch Wasser.

Die erfindungsgemäße Hydrierung wird entweder in Gegenwart von Katalysatoren vorgenommen oder durch andere geeignete Wasserstoffdonore durchgeführt. Als Katalysatoren kommen solche in Frage, die man im allgemeinen für Hydrierungen benutzt wie Raney-Nickel, Platindioxid, Palladium/Kohle usw.; andere geeignete Wasserstoffdonoren sind z. B. Natriumborhydrid oder Natriumcyanoborhydrid.

Die Reaktionstemperaturen können für beide oben genannten Reaktionsstufen in einem großen Bereich variiert werden. Im allgemeinen arbeitet man zwischen 0 und etwa 150°C, vorzugsweise zwischen 20 und 100°C.

Die Umsetzung kann bei den beiden oben genannten Reaktionsstufen bei Normaldruck oder bei erhöhtem Druck durchgeführt werden. Im allgemeinen arbeitet man bei Drucken zwischen 1 und 150 bar.

## Beispiel 1

Eine Lösung von 2 g 5-Amino-5-desoxy-1.2-O-isopropyliden-α-D-glucofuranose in 8 ml 2 N Salzsäure wird 24 Stunden gerührt. Es wird mit 5 ml Wasser verdünnt und nach Zugabe von 0,69 g Triäthylamin und 0,3 g Raney-Nickel 5 Stunden bei 3,5 bar hydriert. Es wird vom Katalysator abfiltriert, im Vakuum eingeengt und zweimal mit Äthanol eingeengt, wobei Kristallisation eintritt. Die Kristalle werden mit Äthanol verrührt, abgesaugt und gut mit Äthanol gewaschen. Man erhält 1,45 g (79,7% der Theorie) 1-Desoxynojirimycinhydrochlorid vom Schmelzpunkt 209 bis 210°C unter Zersetzung.

## Beispiel 2

2 g 6-Amino-6-desoxy-L-sorbofuranose-hydrochlorid-monohydrat (hergestellt aus 6-Amino-2.3-isopropyliden-6-desoxy-α-L-sorbofuranose durch 24stündiges Verrühren in 2 N Salzsäure) werden in 10 ml Wasser gelöst und mit 0,2 g Platindioxid 2 Stunden bei 3,5 bar hydriert. Es wird vom Katalysator abfiltriert, eingeengt, zweimal mit Methanol eingeengt, die erhaltenen Kristalle mit Methanol verrührt und abgesaugt. Man erhält 1,3 g (76% der Theorie) 1-Desoxy-nojirimycin-hydrochlorid vom Schmelzpunkt 210°C unter Zersetzung.

## Beispiel 3

500 mg (2,5 Mol) 1-Desoxy-nojirimycin-hydrochlorid werden im 2 ml Dimethylformid suspendiert. Dann gibt man uner Rühren 0,7 ml (5 mMol) Triäthylamin zu anschließend 5 ml Äthanol. Nach 30 Minuten wird abgesaugt und gut mit Äthanol gewaschen.

Man erhält 385 mg (95% der Theorie) chloridfreies 1-Desoxy-nojirimycin vom Schmelzpunkt 196°C.

## Beispiel 4

### N-Phenyl-1-desoxy-nojirimycin

250 mg 6-Phenylamino-2,3-O-isopropyliden-6-desoxy-α-L-sorbofuranose werden in 1,0 ml 6 n HCl gelöst und 18 Stunden bei 0°C stehengelassen. Es wird mit 3 ml Wasser verdünnt, mit 0,755 ml Triethylamin versetzt und 3 Stunden bei 3 bar $H_2$ mit Raney-Nickel als Katalysator hydriert. Es wird vom Katalysator filtriert, mit Wasser nachgewaschen, mit einem Anionenaustauscher ($OH^{\ominus}$-Form) behandelt, filtriert und eingeengt. Der erhaltene Eindampfrückstand wird massenspektrometrisch untersucht. Man erhält u. a. folgende Massenpeaks:

m/e 239 (Molpeak), m/e 208 (M-$CH_2OH$)
m/e 148 (M-$CH_2OH$-2 CHOH)

## Beispiel 5

Eine Lösung von 1 g 5-Methylamino-5-desoxy-1,2-O-isopropyliden-α-D-glucofuranose vom Schmelzpunkt 127°C in 4 ml 6 n HCl wird 18 Stunden bei 0°C stehengelassen, mit Wasser auf ca. 10 ml verdünnt, mit 2,76 ml Triethylamin versetzt und mit 0,2 g Platinoxid 3 Stunden bei 3,5 bar $H_2$ hydriert. Es

wird filtriert und mit Wasser gewaschen. Die wäßrige Lösung wird auf eine mit einem Kationenaustauscher ($H^{\oplus}$-Form) gefüllte Säule aufgetragen und mit 1%igem wäßrigem Ammoniak eluiert. Das Eluat wird eingeengt, zweimal mit Äthanol aufgenommen und eingeengt. Beim Abkühlen kristalliert die gewünschte Verbindung aus. Sie wird mit Methanol verrührt, abfiltriert und mit Methanol gewaschen. Man erhält 420 mg N-Methyl-1-desoxynojirimycin vom Schmelzpunkt 152° C.

**Patentansprüche**

1. Verfahren zur Herstellung von 1-Desoxy-nojirimycin und N-substituierten Derivaten der Formel

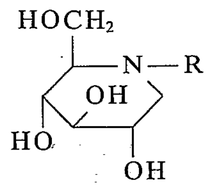

worin R Wasserstoff, Alkyl, Aralkyl oder Aryl bedeutet, dadurch gekennzeichnet, daß man Verbindungen der Formeln

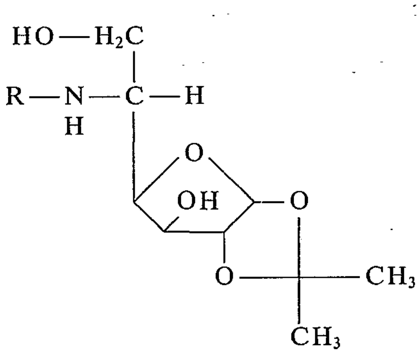

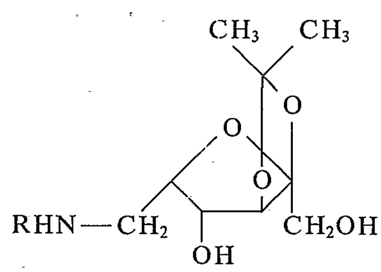

in denen R die oben angegebene Bedeutung hat, durch Behandlung mit Säuren deblockiert und die Zwischenprodukte in Form ihrer Salze isoliert und mit geeigneten Wasserstoffdonoren hydriert oder in einem Eintopfverfahren die Ausgangsverbindungen zuerst durch Behandlung mit Säuren deblockiert und nach gezielter Zugabe von Base direkt hydriert.

2. Verfahren nach Anspruch 1, dadurch gekennzeichnet, daß man mit Salzsäure deblockiert.

3. Verfahren nach Anspruch 1, dadurch gekennzeichnet, daß man 2 N bis 10 N Salzsäure verwendet.

4. Verfahren nach Anspruch 1, dadurch gekennzeichnet, daß man die deblockierten Zwischenprodukte in Form ihrer Salze hydriert.

5. Verfahren nach Anspruch 1, dadurch gekennzeichnet, daß man das Verfahren bei 0 bis 150° C und 1 bis 150 bar durchführt.

**Claims**

1. Process for the preparation of 1-desoxy-nojirimicin and N-substituted derivatives, of the formula

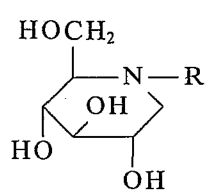

wherein

R denotes hydrogen, alkyl, aralkyl or aryl, characterised in that compounds of the formulae

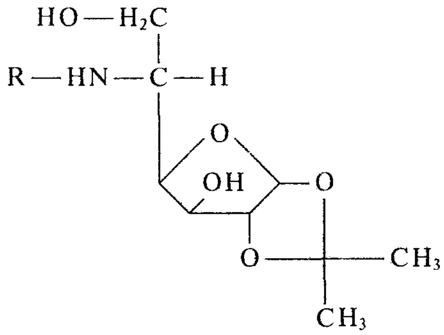

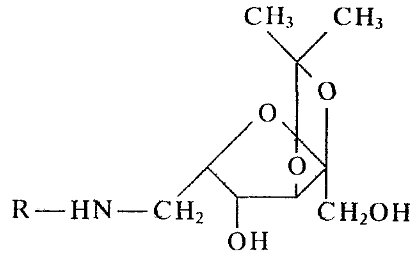

in which

R has the meaning indicated above, are deblocked by treatment with acids and the intermediate are deblocked by treatment with acids and the intermediate products are isolated in the form of their salts and hydrogenated with suitable hydrogen donors, or, in a one-pot process, the starting compounds are first deblocked by treatment with acids and, after controlled addition of a base, hydrogenated directly.

2. Process according to Claim 1, characterised in that the deblocking is carried out with hydrochloric acid.

3. Process according to Claim 1, characterised in that 2 N to 10 N hydrochloric acid is used.

4. Process according to Claim 1, characterised in that the deblocked intermediate products are hydrogenated in the form of their salts.

5. Process according to Claim 1, characterised in that the process is carried out at 0 to 150°C and under 1 to 150 bars.

**Revendications**

1. Procédé de production de 1-désoxy-nojirimycine et de dérvés N-substitués de formule:

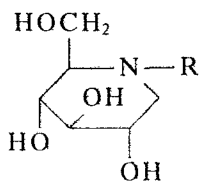

dans laquelle R représente l'hydrogène, un groupe alkyle, un groupe aralkyle ou un groupe aryle, caractérisé en ce qu'on débloque par traitement avec des acides des composés de formules:

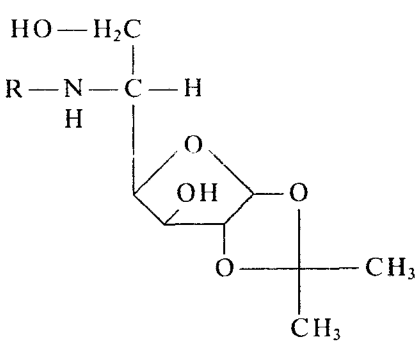

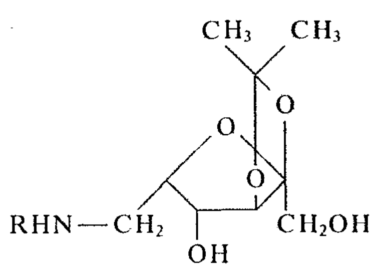

dans lesquelles R a la définition donnée ci-dessus, et on isole les produits intermédiaires sous la forme de leurs sels et on les hydrogène avec des donneurs d'hydrogène convenables ou, dans un procédé direct, on débloque d'abord les composés de départ par traitement avec des acides et on les hydrogène directement après l'addition appropriée d'une base.

2. Procédé suivant la revendication 1, caractérisé en ce qu'on effectue le déblocage à l'acide chlorhydrique.

3. Procédé suivant la revendication 1, caractérisé en ce qu'on utilise de l'acide chlorhydrique 2 N à 10 N.

4. Procédé suivant la revendication 1, caractérisé en ce qu'on hydrogène les produits intermédiaires débloqués sous la forme de leurs sels.

5. Procédé suivant la revendication 1, caractérisé en ce qu'on le met en oeuvre à une température de 0 à 150° C et sous pression de 1 à 150 bars (0,1 à 15 MPa).